Europäisches Patentamt

European Patent Office

Office européen des brevets

(11) Veröffentlichungsnummer: **0 399 096**
**A2**

# (12) EUROPÄISCHE PATENTANMELDUNG

(21) Anmeldenummer: **89118851.8**

(22) Anmeldetag: **11.10.89**

(51) Int. Cl.5: **A61L 29/00**

(30) Priorität: **22.05.89 DE 3916648**

(43) Veröffentlichungstag der Anmeldung:
**28.11.90 Patentblatt 90/48**

(84) Benannte Vertragsstaaten:
**AT BE CH DE FR GB IT LI NL SE**

(71) Anmelder: **Firma Carl Freudenberg**
**Höhnerweg 2-4**
**D-6940 Weinheim/Bergstrasse(DE)**

(72) Erfinder: **Brenner, Otto**
**Bismarckstrasse 38**
**D-6803 Edingen(DE)**
Erfinder: **Josefiak, Christoph, Dr. rer.-nat.**
**Volkerstrasse 3**
**D-6149 Rimbach(DE)**
Erfinder: **Schuhmacher, Günter, Dr. rer.-nat.**
**Steingasse 11**
**D-6940 Weinheim(DE)**

(54) **Schlauch- oder ringförmige, elastische Manschette mit mikrobiziden Eigenschaften für Harnröhrenkatheter.**

(57) Eine schlauch- oder ringförmige, elastische Manschette dient zum Aufziehen auf Harnröhrenkatheter. Sie ist gefertigt aus mikrobizid ausgerüstetem, elastomerem Silikonpolyurethan und sitzt mit radialer Spannung unverrückbar auf dem Kathetermantel auf. Die Manschette besitzt eine sehr hohe Zug- und Reißfestigkeit und hat eine Wandstärke von 0,1 mm. Sie ist 2 bis 10 cm lang und kann unterbrochen in Einzelstücken auf den Katheter aufgezogen werden.

EP 0 399 096 A2

# Schlauch- oder ringförmige, elastische Manschette mit mikrobiziden Eigenschaften für Harnröhrenkatheter

Die vorliegende Erfindung befaßt sich mit einer besonderen Art der mikrobiziden Ausrüstung von Harnröhrenkathetern.

Die zur Vermeidung von Gewebsentzündungen notwendige Ausrüstung von in Körperöffnungen einzuführenden, medizinischen Kathetern mit Metallionen abgebenden, mikrobiziden Wirkstoffen erfolgt auf zwei Wegen:

Entweder werden die Oberflächen des Katheters mit einem antimikrobielle Wirkstoffe enthaltenden Lack behandelt, wie dies in der US-A-4,054,139 oder der US-A-4,612,337 beschrieben ist. Hierzu sind zahlreiche Verfahrensgänge, wie mehrmaliges Quellen, Tränken und Trocknen der Werkstoffe, notwendig.

Ein zweiter Weg zur Ausrüstung besteht darin, den gesamten Katheter aus polymerem Vollmaterial zu fertigen, welches das Mikrobizid enthält, was wegen der verschiedenartigen Oberflächengeometrie der Kathetertypen ein sehr teures und kompliziertes Herstellungsverfahren bedingt. Zudem wird dieses Verfahren erheblich verteuert durch die benötigte große Menge an mikrobiziden, Metallionen abgebenden Materialien.

Die Aufgabe der vorliegenden Erfindung besteht darin, eine antimikrobielle Ausrüstung für Harnröhrenkatheter anzugeben, die mit wenigen Verfahrensschritten und geringem Materialbedarf herstellbar und die vor allem universell für alle Kaliber und Formen dieser Katheter anwendbar ist, wobei eine umfassende und dauerhafte keimtötende Wirksamkeit mit Sicherheit gewährleistet sein muß. Die Manschette muß sehr zug- und reißfest sein und zur Vermeidung von Verletzungen eine Oberflächenrauhigkeit unter 6 $\mu$m besitzen.

Als Lösung dieser Aufgabe wird eine schlauch- oder ringförmige, elastische Manschette mit den in Anspruch 1 genannten Merkmalen vorgeschlagen. Ein vorteilhaftes Verfahren zur Verwendung dieser Manschette wird ebenfalls beansprucht.

Der Anmelderin sind vor dem Prioritätstag keine solchen Manschetten bekannt gewesen.

Die erfindungsgemäße Manschette wird als schlauchförmige Hülse auf konventionelle Katheter aufgeschoben. Sie besteht dazu aus einem sehr dünnen, elastomeren Kunststoff, welcher die in der Medizintechnik bekannten, bakterizide Metallionen abgebenden Substanzen eingebettet enthält, so zum Beispiel $Ag^+$, $Au^+$, $Cu^{++}$ oder andere Metallionen enthaltende Metallsalze, deren Oxide und Carbide sowie metallorganische Verbindungen. Besonders bewährt hat sich Silbersulfat.

Der elastomere Kunststoff ist silikonmodifiziertes Polyurethan, gemäß Patentanmeldung P 37 25 728, wo auch seine Herstellung geschildert ist: Dieser Werkstoff enthält in seiner Hauptkette zu weniger als 50 Gew.-% ein organisches Silikonpolymeres mit einem Molekulargewicht von 500 bis 10 000. Der gesamte Querschnitt des Elastomeren ist mit einer Metallionen abgebenden Substanz als alleinigem Wirkstoff in räumlich gleichmäßiger Verteilung und in Mengen von 1 bis 15 Gew.-% ausgefüllt, bezogen auf die Gesamtmasse des Elastomeren.

Für Katheter geeignete, erfindungsgemäße Manschetten sind durch Schlauchextrusion aus diesem Werkstoff einfach herstellbar. Dabei werden Hülsen erhalten, die bei einer Zugfestigkeit von 20 MPa und einer Reißdehnung von 500 % eine Shore-A-Härte zwischen 80 und 95 und eine Oberflächenrauhigkeit unter 6 $\mu$m aufweisen.

Am Markt befindliche Harnröhrenkatheter werden in drei Stärkeklassen unterteilt:

26 bis 19 Charier-Einheiten (= 8,7 bis 6,3 mm Durchmesser)

18 bis 13 Charier-Einheiten (= 6,0 bis 4,3 mm Durchmesser)

12 bis 8 Charier-Einheiten (= 4,0 bis 2,7 mm Durchmesser)

Es genügt, Manschetten mit drei verschiedenen Innendurchmessern, entsprechend den drei Charier-Klassen der Katheter, herzustellen, wobei der Innendurchmesser der Manschette stets sich zum Außendurchmesser des Katheters verhalten muß wie 1 : 1,2 bis 1 : 1,8; die erfindungsgemäßen Manschetten werden also so konfektioniert sein müssen, daß ihr Innendurchmesser zwischen 2,0 und 8,0 mm liegt, um in jedem Fall mit radialer Vorspannung auf den jeweiligen Harnröhrenkatheter aufziehbar zu sein. Somit haftet die Manschette unverrückbar auf diesem.

Alle diese Materialdaten gewährleisten, daß trotz Spannung keine Beschädigung der Manschette beim Aufziehen und beim Gebrauch des Katheters auftreten.

Die Wandstärke des elastomeren Werkstoffes darf nur 0,1 mm betragen, mit einer Toleranzgrenze von ± 5 %, um die Flexibilität des Katheters nicht durch die Umhüllung nachteilig zu erniedrigen.

Das Schlauchmaterial wird nach der Extrusion in 2 bis 10 cm lange Stücke zerschnitten, welche sodann bei einer Temperatur zwischen 180 und 220 °C umgeschmolzen werden, um eine Abrundung der Kanten an den Manschetten-Enden zu erzielen. Diese Maßnahme vermindert die Verletzungsgefahr durch scharfe Übergänge vom Manschetten- zum Kathetermaterial, wenn der Katheter in der Körperhöhle verschoben wird.

Eine besonders wirtschaftliche, weil materialsparende Anwendungsweise der erfindungsgemäßen Manschette besteht darin, daß man mit ihr insgesamt nur mindestens 50 % der Länge des Katheters überzieht: Dazu werden, nach Auswahl des geeigneten Innendurchmessers, axial zueinander zwischen 0,1 und 3 cm beabstandet, 5 cm lange Manschettenringe unter Vorspannung aufgezogen. Zweckmäßigerweise wird man zur Sicherheit Abstände zwischen 0,2 und 0,4 cm vorsehen. Neben der Materialersparnis ergibt sich der Vorteil einer größeren Flexibilität des Katheters, ohne daß Einbußen bezüglich der bakteriziden Wirkung in Kauf genommen werden müßten; die Ausbildung eines "Hemmhofes" für das Mikroben- und Bakterienwachstum durch die Metallionen aus dem benachbarten Manschetten-Material überbrückt wirksam die freiliegenden Oberflächen des eigentlichen Harnröhrenkatheters.

Zum Aufziehen der Manschetten auf die Katheter kann eine Aufziehhilfe von konischer Form verwendet werden. Dabei ist es zweckmäßig, pro Charier-Klasse jeweils einen Konus aus PTFE zu verwenden, dessen größter Durchmesser größer ist als der Außendurchmesser des größten Katheters der jweiligen Klasse. Der kleinste Durchmesser des Aufziehkonus' sollte etwa halb so groß sein wie derjenige der gegenüberliegenden Seite.

Die Länge der Aufziehhilfe ist zweckmäßig 40 bis 50 cm. Über das verjüngte Ende wird die Manschette aufgezogen und dabei auf ca. 80 % ihrer lichten Weite gedehnt. Der Konus wird nun mit seinem dicken Ende an den Katheter angelegt, wonach die Manschette in Richtung des Katheters von der Aufziehhilfe abgestreift werden kann. Deren Konusform gewährleistet, daß die Manschette beim Aufziehen auf den Katheter stets über ihren gesamten Umfang gleichmäßig gedehnt wird und somit ihre schonende Handhabung gewährleistet ist.

Für alle Ausgestaltungen der Erfindung besteht der Vorteil, daß die Produktion von Harnröhrenkathetern aus zwar physiologisch unschädlichen, jedoch biologisch inaktiven und daher kostengünstigen Materialien erfolgen kann, ohne der antimikrobiellen Ausrüstung Beachtung schenken zu müssen. Der Hersteller ist so in bezug auf die Auswahl von Materialdaten wie Flexibilität und Biegebruch völlig unbehindert. Mit den erfindungsgemäße Manschetten ist es dabei möglich, nachträglich in einfachster Weise mit nur drei Konfektionsgrößen die drei bedeutenden Charier-Klassen an Harnröhrenkathetern zuverlässig antimikrobiell auszurüsten.

Die folgende Tabelle zeigt beispielhaft die mechanische Beanspruchung von drei Konfektionsgrößen der erfindungsgemäßen Manschette, die jeweils auf die bezüglich ihrer Charier-Klasse zugehörigen Harnröhrenkatheter unter Vorspannung aufgezogen werden.

| Charier-Klasse Katheter, Einheiten | Innendurchmesser der Hülse, mm | Dehnung der Hülse | |
|---|---|---|---|
| | | maximal | minimal |
| 26 bis 19 | 5,8 | 50 % | 9 % |
| 18 bis 13 | 4,0 | 50 % | 8 % |
| 12 bis 8 | 2,5 | 60 % | 8 % |

Die Beanspruchung auf Dehnung der Manschetten liegt also stets weit unter ihrer Höchstzugdehnung und somit im beschädigungsfreien Bereich.

## Ansprüche

1. Schlauch- oder ringförmige, elastische Manschette zum Aufziehen auf Harnröhrenkatheter, bestehend aus einem Silikonpolyurethan-Elastomer, in dessen Hauptkette zu weniger als 50 Gew.-% ein organisches Silikonpolymeres mit einem Molekulargewicht von 500 bis 10 000 vorhanden ist und in dessen gesamte Querschnitt eine Metallionen abgebende Substanz als alleiniger Wirkstoff in räumlich gleichmäßiger Verteilung und in Mengen von 1 bis 15 Gew.-%, bezogen auf die Gesamtmasse des Elastomeren, eingelagert ist, wobei die Manschette
eine Shore A- Härte zwischen 80 und 95 (DIN 53 505),
eine Wandstärke von 0,1 mm ± 5 %,
eine Länge zwischen 2 und 10 cm und

einen Innendurchmesser von 2,0 bis 8,0 mm besitzt.

2. Verfahren zum Ausrüsten eines Harnröhrenkatheters mit einer Manschette gemäß Anspruch 1, dadurch gekennzeichnet, daß man eine Manschette auswählt, deren Innendurchmesser sich zum Außendurchmesser des Katheters verhält wie 1 : 1,2 bis 1 : 1,8, und daß man mehrere 5 cm lange Manschettenstücke hintereinander unter Vorspannung aufzieht mit axialen Abständen zueinander zwischen 0,1 und 3 cm, bis mindestens 50 % der Länge des Katheters vom Hülsenmaterial bedeckt sind.